(19) Europäisches Patentamt
European Patent Office
Office européen des brevets

(11) EP 1 909 102 A1

(12) **EUROPEAN PATENT APPLICATION**

(43) Date of publication:
**09.04.2008 Bulletin 2008/15**

(51) Int Cl.:
***G01N 33/50*** *(2006.01)*

(21) Application number: **07019573.0**

(22) Date of filing: **05.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **06.10.2006 US 849784 P**

(71) Applicant: **c-a-i-r biosciences GmbH**
**72076 Tübingen (DE)**

(72) Inventors:
- **Schett, Georg**
  **91080 Marloffstein (DE)**
- **Albrecht, Wolfgang**
  **89075 Ulm (DE)**
- **Laufer, Stefan**
  **72076 Tübingen (DE)**

(74) Representative: **Reitstötter - Kinzebach**
**Patentanwälte,**
**Sternwartstrasse 4**
**81679 München (DE)**

(54) **Method for subgroup analysis in subjects having or being suspected of having inflammatory disease, use of anti-p38mapk antibodies, kits and their use**

(57) The present invention relates to a method of determining whether a subject having or suspected of having inflammatory disease may potentially benefit from treatment with a p38 mitogen-activated protein kinase (p38MAPK) inhibitor, which method comprises
(I) obtaining an inflammatory tissue sample from the subject; and
(II) determining whether p38MAPK, in particular p38MAPKa, is expressed in the tissue sample.

The present invention further relates to the use of an antibody against the p38MAPK protein for determining whether a subject having or suspected of having inflammatory disease may benefit from treatment with a p38 mitogen-activated protein kinase (p38MAPK) inhibitor; kits for determining from an inflammatory tissue sample whether a subject having or suspected of having inflammatory disease may benefit from treatment with a p38 mitogen-activated protein kinase (p38MAPK) inhibitor; and to the corresponding use of said kit.

EP 1 909 102 A1

## Description

The present invention relates to a method for subgroup analysis in subjects having or being suspected of having inflammatory disease, e. g. rheumatoid arthritis, based on the measuring of p38MAPK expression in order to predict the potential benefits of p38MAPK inhibitor treatment, and to uses of anti-p38MAPK antibodies for such a subgroup analysis.

Inflammation is the first response of the vertebrate immune system to infection or irritation and is characterised by the following quintet: redness ("rubor"), heat ("calor"), swelling ("tumor"), pain ("dolor") and dysfunction of the organs involved ("functio laesa"). The first four characteristics have been known since ancient times and are attributed to Celsus; "functio laesa" was added to the definition of inflammation by Rudolf Virchow in 1858. The inflammatory reaction can be induced by a variety of agents, most of which are either microbiological in nature (e. g. bacterial lipopolysaccharide) or inadvertently mimic microbiological components. Thus, inflammation is primarily a reaction that aims at elimination of a potentially pathogenic microorganism from a site of infestation, even at the price of locally damaging the host's own tissue, which may later lead to scarring.

A variety of cells are involved in the inflammatory process, beginning with the initial sensing of the inductive agent which leads to the release of an array of chemical mediators which serve as chemoattractants for immunocytes and locally increase vascular permeability and blood coagulability, thereby leading to the aforementioned phenomena (e. g. the swelling due to increased vascular permeability distends the tissues, compresses nerve endings, and thus causes pain). Among these mediators, tumour necrosis factor alpha (TNF$\alpha$) and interleukins 1 and 6 (IL-1 and IL-6) are considered as the most important. Additionally, endothelial cells are stimulated to up-regulate cell surface molecules such as VCAM-1, ICAM-1, E-selectin, and L-selectin, which enhance attachment and extravasation into the surrounding tissue of immunocytes. At the same time, immunocytes, in particular macrophages and granulocytes, are stimulated to perform phagocytosis (enabling them not only to directly devour and destroy microorganisms but also to present specific peptides for the subsequent induction of the secondary, specific immune response) and to release reactive radicals and other microbicidal, but also potentially tissue-damaging, substances. Granulocytes, in particular, are apt to perform a "respiratory burst" providing a self-destructive flash of reactive oxygen species such as superoxide radicals and hydrogen peroxide ("suicide bombers"). In many cases, the harm done to the host's tissue by the release of these aggressive compounds exceeds the direct damage done by the infesting agent, in particular if the latter is indeed non-microbial in nature.

If the injurious agent cannot be removed, chronic inflammation is likely to ensue, lasting for a period of days, months or even years and characterised by a dominating presence of macrophages in the injured tissue, which still extravasate into the tissue as described above (in particular using ICAM-1 and VCAM-1) and keep on releasing strongly microbicidal agents including reactive oxygen species that are injurious to the host's own tissues. Thus, chronic inflammation usually entails tissue destruction.

P38-mitogen-activated proteins (p38MAPKs) are members of a larger group of serine-threonine protein kinases, which allow the transduction of extracellular stress signals to the cell nucleus. MAPKs are reporters of change of the extracellular milieu, which lead to cellular responses allowing an adaptation towards changed physiological and pathological circumstances, in particular reactions induced by signalling molecules. p38MAPK is considered as a common pathway of inflammatory stimuli such as tumour necrosis factor (TNF) and interleukin-1 (IL-1) as well as of environmental stress, such as heat stress, osmotic shock, ultraviolet light and cytotoxic chemicals. Thus, p38MAPKs functions to transmit an "emergency switch" signal, which leads to a complex cellular response by turning on various target genes of transcription factors, cytokines and their surface receptors. In the quest for new treatments which aim at shutting down inadequate inflammatory responses such as those involved in rheumatoid arthritis, these signal transmission proteins are therefore considered as promising targets for future therapeutic compounds.

P38MAPK has been first defined upon stimulating cells with lipopolysaccharide (LPS), leading to the discovery of the $\alpha$-isoform of p38MAPK (also known as SAPK2a, "stress-activated protein kinase 2a", or CSBP1, "CSAID-binding protein 1 "; further aliases comprise RK, EXIP, Mxi2, PRKM14, PRKM15 and MAPK14) which was shown to be involved in the synthesis of proinflammatory cytokines TNF and IL-1.

Next, the $\beta$-isoform of p38MAPK (also known as SAPK2b) was described, which shares a 75 % structural homology with p38MAPK$\alpha$ and is also activated by proinflammatory cytokines as well as by environmental stress. Later, in myocytes there was a third isoform found, p38MAPK$\gamma$ (also known as SAPK3 or ERK6) whose homology with p38MAPK$\alpha$ amounts to 63 %. Finally, p38MAPK$\delta$ (also known as SAPK4) was identified as a fourth isoform of p38MAPK, sharing 57 % homology with p38MAPK$\alpha$, and, like all other isoforms of p38MAPKs, was responsive to inflammatory and toxic environmental signals.

Importantly, p38MAPKs play a major role in proinflammatory cytokine production by activating transcription factors binding to the promoter regions of many cytokines, including TNF, IL-1 and IL-6, and thus represent a pivotal part of the inflammatory mechanism. Regulation of these cytokines is of key importance in the balance of pro- and anti-inflammatory mechanisms, which are important in determining whether a given environmental trigger results in a physiological self-limited inflammatory response correctly ending shortly after elimination of the injurious agent or results in sustained inflammatory activity potentially leading to chronic inflammatory disease and tissue damage. In particular, they have been shown to participate in metalloproteinase upregulation and osteoclast activation, thereby directly promoting not only inflammation but also bone erosion in the affected joints. Thus, p38MAPKs are crucial to several aspects of inflammatory disease.

Rheumatoid arthritis is a typical and clinically highly relevant example of an inflammatory disease, which is characterized by sustained synthesis of proinflammatory cytokines. It is therefore not surprising that p38MAPK is activated in chronic inflammation of humans, and in particular in rheumatoid arthritis. It was demonstrated before that selective blockade of TNF, IL-1 or IL-6 can control disease by inhibiting inflammation of the joints and structural damage, indicating that imbalanced cytokine production is one of the key events in the genesis and/or maintenance of rheumatoid arthritis.

Being regulators of cytokine production as well as important intracellular signalling pathway components of the same cytokines, the role of p38MAPKs in the context of cytokine production, which they both sense and stimulate, is crucial and suggestive of a potential feedback loop. This gave the rationale to develop specific synthetic inhibitors for the termination of states of chronic inflammation. Such compounds indeed were shown to have antiinflammatory properties in experimental animal models of arthritis, and they have also passed early phase clinical trials.

It has been claimed that p38MAPK inhibitors were of benefit in treating a large variety of disorders. However, a beneficial effect in inflammatory disease was demonstrated only for a part of the subjects treated. In the VeRA trial, which was conducted to evaluate treatment effects in terms of characteristic manifestations of the disease such as tender joints, swollen joints, morning stiffness and disease activity in a cohort of 315 patients with moderate to severe rheumatoid arthritis, approximately 40 % of patients were responsive to treatment with the p38MAPK inhibitor VX-702, compared to a placebo effect of about 30 %, arguing for a biochemical net response rate of about 28 % according to a Monte Carlo simulation of the distribution of the effects. So far, no explanation for this evident dichotomy between responders and non-responders has been proposed yet.

Thus, it was an object of the present invention to provide a method for subgroup analysis to individually determine whether a given subject suffering from inflammatory disease, in particular from rheumatoid arthritis, may potentially benefit from the administration of a p38MAPK inhibitor.

Surprisingly, it was found that the tissue expression profiles of the four different isoforms of p38MAPK differ widely among subjects which present with inflammatory disease, even with clinically indistinguishable manifestations of inflammatory disease, although the causal involvement of the p38MAPKs, where they are present, has been proven, and that in particular the expression of p38MAPK$\alpha$, previously shown to be an important mediator of inflammatory processes, in synovial fibroblasts and macrophages may be either present or absent in clinically manifest inflammatory disease such as rheumatoid arthritis; in other ways, that inflammatory disease may, but need not, be mediated by a signalling pathway involving p38MAPK and in particular p38MAPK$\alpha$.

Subjects presenting with inflammatory disease may therefore be classified as having either high-p38MAPK$\alpha$ or low-p38MAPK$\alpha$ inflammatory disease. The former (the "textbook case" of inflammation) are the ones who can be expected to particularly benefit from the administration of p38MAPK inhibitors, in particular p38MAPK$\alpha$ inhibitors. Without wishing to be limited by theory, it will be assumed that in the case of the newly-described low-p38MAPK inflammatory disease the state of inflammation may be induced and/or maintained by a mechanism or mechanisms independent of p38MAPK, in particular a mechanism independent of p38MAPK$\alpha$, and that other treatments for this particular subgroup, rather than inhibition of p38MAPK, should therefore be sought, whereas in the case of high-p38MAPK inflammatory disease inflammation is maintained essentially by a p38MAPK-dependent mechanism and inhibition of p38MAPK, alone or in combination with other drugs and/or treatments, may therefore be beneficial.

Thus, the present invention relates to a method of determining whether a subject having or suspected of having inflammatory disease may potentially benefit from treatment with a p38 mitogen-activated protein kinase (p38MAPK) inhibitor, which method comprises

(I) obtaining an inflammatory tissue sample from the subject; and
(II) determining whether p38MAPK, in particular p38MAPK$\alpha$, is expressed in the tissue sample.

The expression of the p38MAPK, in particular p38MAPK$\alpha$, indicates the subject's potential benefits, and administration of p38MAPK inhibitors can be restricted to the p38MAPK-expressing subgroup of patients where it can be expected to be effective.

Being capable of determining the subject's potential benefits from treatment with a p38 mitogen-activated protein kinase (p38MAPK) inhibitor has several advantages.

First, p38MAPKs are involved in a wider range of cellular processes than only the regulation and signalling of proinflammatory cytokines. So far, control of the cell cycle and apoptosis, angiogenesis, regulation of oxidative processes and chemotaxis have been reported to be influenced by p38MAPKs, each of these aspects in fact comprising a complex network of interactions and feedbacks. Second, many existing p38MAPK inhibitors, such as most pyrimidinylimidazole

p38MAPK inhibitors, are burdened with a propensity for severe side effects, in particular hepatotoxicity, presumably by causing interference with liver cytochrome P450, as mentioned above; but even the more specific isoxazole-based inhibitors, discussed further below, will still, by virtue of the manifold contributions of p38MAPKs to intracellular signalling, inevitably influence a wide variety of complexly interwoven signalling pathways. It is therefore to be expected that these intrinsic side-effects will also burden newer inhibitors developed in the future. Thus, pharmacological inhibition of p38MAPK will result in generally pleiotropic effects, many of which may be unwanted or even dangerous, even if these inhibitors are pharmacologically restricted, by use of a drug targeting system or otherwise, to the tissue to be treated.

Because of the aforesaid pleiotropic effects and the unwanted side effects likely to result from them, such as gastroenteritis, nausea and vomiting, rash, renal impairment, and generally increased susceptibility to infections, it is advantageous to distinguish likely responders from likely non-responders before the initiation of treatment with p38MAPK inhibitors. Hence, one advantage of the present invention is to be able to restrict p38MAPK inhibitor treatment to cases of verifiable need.

Preferably, the estimation of an individual subject's potential benefits comprises (III) the use of a correlative function or table of any kind giving the likelihood of benefits to be expected from treatment with p38MAPK inhibitors, preferably with specific p38MAPKα inhibitors, for a subject with the specific level of p38MAPK, in particular p38MAPKα, expression determined in (II). Expediently, the function or table will reflect experience from previous clinical trials.

Generally, the function or table will take the form

$$B = F( E ) \qquad (1),$$

wherein

E is a matrix or profile of p38MAPK expression, preferably relative expression in comparison to healthy controls,

B is the probability of beneficial effects, and

F is the correlative function incorporating previous clinical experience.

It will be appreciated that F does not have to be an arithmetic function but may be a plain list of clinical findings. E may be the absolute or relative overall expression of p38MAPK; the absolute or relative expression of p38MAPKα; the absolute or relative expression of p38MAPKα, p38MAPKβ, p38MAPKγ and p38MAPKδ, determined individually; the phosphorylation level of any of these enzymes; or any combination of the said values which have been empirically found to be useful in attributing to a given subject either a high or a low value of B, wherein a high value of B ($B > 50\ \%$) is considered as implicating a recommendation to use p38MAPK inhibitors in the treatment of a subject exhibiting such a B value.

According a more particular embodiment, the function or table will take the general form

$$B = F( E, C ) \qquad (2),$$

where B, F and E have the meaning as described above and

C is a matrix or profile of additional relevant characteristics of the individual subject, such as further individual and/or clinical parameters, concomitant treatment and the like. It is to be expected that this will allow for more reliable estimation of the likelihood of benefits from treatment, once the required clinical studies have been completed.

Thus, in a preferred embodiment of the invention E is selected from the group consisting of

[XSa];
[ XSα / X0α ] ;
[ PSα ] ;
[ PSα/P0α ];
[ XSα ; PSα ] ;
[ XSα+XSβ+XSγ+XSδ ] ;
[ (XSα+XSβ+XSγ+XSδ) / (X0α+X0β+X0γ+X0δ) ] ;
[ PSα+PSβ+PSγ+PSδ ] ;
[ (PSα+PSβ+PSγ+PSδ) / (P0α+P0β+P0γ+P0δ) ] ;
[ XSα ; XSα+XSβ+XSγ+XSδ ];
[ (XSα+XSβ+XSγ+XSδ) / (X0α+X0β+X0γ+X0δ)] ;
[ PSα+PSβ+PSγ+PSδ] ; and
[ (PSα+PSβ+PSγ+PSδ) / (P0α+POβ+P0γ+P0δ) ] ,

wherein XAn is the expression of p38MAPKn in the tissue of interest of A, where A is either a subject S having or being

suspected of having inflammatory disease, in particular rheumatoid arthritis, or a healthy control 0 not having inflammatory disease, and PAn is the level of phosphorylation of the same enzyme.

In a preferred embodiment of the invention, C is selected from the group consisting of

[ gender; age ; duration of disease ; blood levels of C-reactive protein ; erythrocyte sedimentation rate ; presence of erosive disease ; concomitant DMARD medication ; concomitant glucocorticoid medication ]

and any of its subsets, preferably any of its non-empty subsets.

More preferably, C is the matrix: [ Gender; age ; duration of disease ; blood levels of C-reactive protein ; erythrocyte sedimentation rate ; presence of erosive disease ; concomitant DMARD medication ; concomitant glucocorticoid medication ].

As used herein, a "p38MAPK" is a serine/threonine protein kinase comprising an amino acid sequence with an overall sequence similarity of at least 50 %, preferably at least 60 %, more preferably at least 70 % and most preferably at least 99 % with any serine/threonine protein kinase selected from the group consisting of p38MAPK$\alpha$, p38MAPK$\beta$, p38MAPK$\gamma$ and p38MAPK$\delta$, preferably with p38MAPK$\alpha$, more preferably with human p38MAPK$\alpha$ and most preferably with the sequence as laid down in SEQ ID NO:1.

As used herein, a "p38MAPK$\alpha$" is a serine/threonine protein kinase comprising an amino acid sequence with an overall sequence similarity of at least 75 %, preferably at least 90 %, more preferably at least 95 % and most preferably at least 99 % with any mammalian p38MAPK$\alpha$, more preferably with human p38MAPK$\alpha$ and most preferably with the sequence as laid down in SEQ ID NO:1.

A "p38MAPK inhibitor" is a substance which is capable of significantly reducing, under the conditions of interest, the enzymatic activity of a p38MAPK. Efficient p38MAPK inhibitors, in particular such as belong to the classes of 4,5-diarylimizadoles, 2,4,5-triarylimidazoles and 3,4,5-triarylimidazoles, preferably pyridinylimidazoles, such as pyridinylimidazoles containing pyridinyl-4-yl and 4-fluorophenyl groups, have been described in the art.

Without wishing to be bound by theory, these substances are assumed to block the ATP binding site of the protein kinase by formation of a hydrogen bond between a particular amide group of the protein backbone and the pyridine nitrogen atom of the inhibitor after insertion of the inhibitor into a hydrophobic pocket. The requirement for exact steric matching in this interaction allows for specificity.

Such p38MAPK inhibitors and methods of synthesizing them have been described in the art and are summarized, e. g., in Wagner et al. 2000 (Arch. Pharm. Pharm. Med. Chem. 333 Suppl. 1, 2000: 97). For example, USP 4’585’771 (Klose et al., 1984) discloses 2-heteroaryl-4,5-diphenylimidazoles having anti-inflammatory and antiallergic activity, and WO 88/01167 (Bender et al., 1986) discloses modulation of IL-1 secretion by human monocytes using 4,5-diaryl-imidazoles, e. g. 2-thio-4-(4-fluorophenyl)-5-(4-pyridyl)-imidazote. USP 4’461’298, USP 4’528’298 (Niedballa & Böttcher, 1983) and USP 4’402’960 (Niedballa & Böttcher, 1981) disclose 2-heteroaryl-4,5-diheteroaryl-imidazoles, e. g. 2-pyridyl-4,5-diheteroaryl-imidazoles, also having anti-inflammatory and antiallergic activity. USP 4’461’770 (Ferrini et al., 1981), USP 4’608’382 (Ferrini et al., 1984) and USP 4’584’310 (Ferrini et al., 1984) disclose 2-alkylthio-, 2-alkylsulfinyl- and 2-alkylsulfonyl-4-aryl-5-heteroaryl-imidazoles having anti-inflammatory activity. DE 198 42 833 and WO 00/17192 (Laufer et al., 1999) relate to 2-phenylalkylthio-4-heteroaryl-5-phenyl-imidazoles acting as cytokine release inhibitors. WO 95/00501 discloses imidazoles having cyclooxygenase inhibitor activity. Boehm et al. (J. Med. Chem. 39, 1996: 3929 - 3937) discloses the inhibitory effects of substituted imidazoles such as 4-aryl-5-pyridyl-imidazoles on cytokine secretion, associated with a low inhibitory effect on 5-lipoxygenase and cylooxygenase, and in particular the cytokine release inhibitor effect of 2-(4-methylsulfinylphenyl)-4-(4-fluorphenyl-5-pyrid-4-yl)-imidazole. WO 99/03837 (Wachter et al., 1998) and WO 93/14081 (Adams et al., 1993) relate to 2-substituted imidazoles, in particular to 2,4,5-triaryl-imidazoles, inhibiting synthesis of a number of proinflammatory cytokines. USP 4’440’776 (Niedballa & Böttcher, 1982), USP 4’355’039 (Niedballa & Böttcher, 190) and USP 4’269’847 (Niedballa & Böttcher, 1979) disclose imidazoles with anti-inflammatory and antiallergic effects. WO 96/03387 (Weier et al, 1995), WO 02/066458 (Laufer, 2002), WO 03/097633 (Laufer, 2003) and WO 2004/018458 (Laufer, 2004) relate to 2-thio-imidazoles with immunomodulatory and cytokine release inhibiting properties, in particular to 2-thio-4-pyridinyl=5-(4-fluorophenyl)-imidazoles which were active in the suppression of TNFa and IL-1$\beta$ synthesis. WO 03/097633 in particular discloses a number of 2-alkylthio-4-(pyridinyl-4-yl)-5-(4-fluorophenyl)-imidazoles with an affinity for p38MAPK$\alpha$ in excess of $10^{-6}$ M, such as 2-(2-chloroethylsulfanyl)-3-methyl-4-(pyridinyl-4-yl)-5-(4-fluorophenyl)-imidazole. Likewise, WO 2004/018458 discloses a number of imidazoles such as 2-methylsulfanyl-4-(2-phenylethalamin-pyridinyl-4-yl)-5-(4-fluorophenyl)-imidazole with an affinity for p38MAPK$\alpha$ in excess of $10^{-6}$ M. Further 2-thio-imidazoles have been described e. g. in EP 0 004 648 (Ferrini et al., 1979); EP 0 005 545 (Niedballa & Böttcher, 1979); EP 372 445 (Billheimer et al., 1989); USP 5’364’875 (Wilde, 1992); USP 4’190’666 (Cherkofsky et al., 1978), GB 1 155 580, JP 01-010-467, SU 1 415 725 (Vinogradow et al., 1986); Liverton et al.1999 (J. Med. Chem. 42, 1999: 2180 - 2190); Mustafa et al. 1972 (J. prakt. Chem. 314, 1972: 785 - 792); Gupta et al. 1982 (Indian J. Chem., 22B(3), 268 - 269); Wilde et al. 1995 (Bioorganic & Medicinal Chem. Lett. 5(2), 1995: 177 - 180); Salama et al. 1988 (Phosphorus & Sulfur 135(1-2), 1988: 83-88); Beach et al. 1992 (Arch. Biochem. Biophys. 297, 1992: 258 - 264); Maduskuie et al. 1995 (J. Med. Chem. 38, 1995: 1067 - 1083); Mioston̉ et al. 1998 (Helv. Chim. Acta 81, 1998: 1585 - 1595); and Mioston̉ et al. 1999 (Helv. Chim. Acta 82, 1999: 290 - 296).

EP 372 445 in particular discloses 2-alkoxy- and 2-alkylthio-3,4,5-phenyl-imidazoles, and USP 5'364'875 relates to imidazoles having a 2-substituent comprising a polycyclic aryl group. Furthermore, WO 2004/110990 discloses bicyclic imidazoles (imidazooxazoles and imidazothiazoles), in particular bicyclic imidazoles with fluorophenyl and substituted pyrimidyl moieties, having p38MAPK inhibitory properties and anti-inflammatory effects, some of which also possess an affinity for p38MAPK$\alpha$ in excess of $10^{-6}$ M, e. g. 4-(4-fluorophenyl)-5-(2-[cyclopropyl-amino]-pyrimidyl-4-yl)-imidazooxazole ($IC_{50}$ for p38MAPK$\alpha$ = $4.4 \times 10^{-10}$ M). Laufer et al. 2002, Angew. Chem. Int. Ed. 41, No. 13, 2002: 2290 - 2293, review recent information on the subject of 4-(4-pyridyl)-5-(4-fluorphenyl)-imidazoles, suggesting distinct roles in p38MAPK binding for the two aromatic residues and the nitrogen atoms of the imidazole ring.

Preferred inhibitors are inhibitors of the general formula (I)

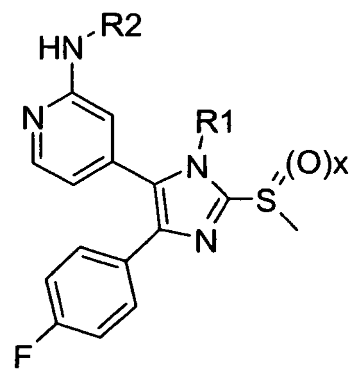

(I)

wherein
x is 0, 1 or 2;
R1 is hydrogen, methyl or $-(CH_2)_2-O-CH_3$; and
R2 is alkyl, in particular $C_1$-$C_6$ alkyl, e. g. methyl, ethyl, propyl or isopropyl; or cycloalkyl, in particular $C_3$-$C_7$ cycloalkyl, e. g. cyclopentyl or cyclohexyl; or -C(O)-R3,
wherein
R3 is alkyl, in particular $C_1$-$C_6$ alkyl, e. g. methyl, ethyl, propyl or isopropyl; or cycloalkyl, in particular $C_3$-$C_7$ cycloalkyl, e. g. cyclopentyl or cyclohexyl.

In particular the following compounds are currently being investigated for their inhibitory effects on p38MAPK: 2-(4-sulfinylmethyl)-4-(4-fluorophenyl)-5-(4-pyridyl)-imidazole (SB-205380), 2-(4-sulfinylmethyl)-4-(4-fluorophenyl)-5-(4-pyridyl)-pyrrole ("compound A") and 1-(4-piperidyl)-4-(4-fluorophenyl)-5-(4-pyridyl)-imidazole (VK-19911) (II):

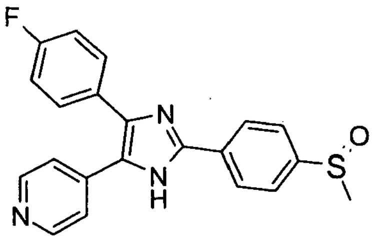

SB-203580

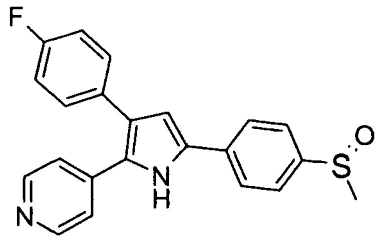

compound A

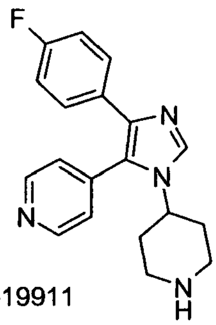

VK-19911

(II)

Among these, the p38MAPK affinity of compounds comprising an imidazole core is markedly stronger (by several orders of magnitude) than that of compounds comprising a pyrrole core, suggesting an important role of the 3-N in target binding. The person skilled in the art will appreciate that different substituents may also lead to different isoform specificity. An overview of the substrate preferences of p38MAPK$\alpha$ is given by Laufer et al. 2006 (Chem. Med. Chem. 1 : 197 - 206).

Thus, pyridinylimidazoles containing a 4-fluorophenyl group in particular are selective inhibitors for p38MAPK$\alpha$, wherein the aforesaid particular amino acid is a methionine residue. The structure of the essential groups (pharmacophores) of these inhibitors is thought to be mimicked by other molecules, in particular that of the novel class of substituted isoxazoles, which were reported to combine selective p38MAPK$\alpha$ inhibition with considerably lower unspecific toxicity (Laufer et al. 2006, Chem. Med. Chem. 2, 197 - 207). In particular, 3,4- and 4,5-disubstituted isoxazoles, preferably isoxazoles substituted with one haloaryl and one pyridinyl group, more preferably isoxazoles substituted with one 4-fluorphenyl and one 4-pyridinyl group, the latter optionally substituted with 3-halo or 3-alkoxy, are potent p38MAPK inhibitors with little inclination to interference with cytochrome P450 (CYP) and ensuing hepatotoxicity as it was described for the older inhibitor class of pyridinylimidazoles.

Examples for non-imidazole-based inhibitors comprise 1-(4-pyridyl)-2-(4-fluorophenyl)-4-(4-chlorophenyl)-furan, 1-(4-fluorophenyl)-2-(4-pyridyl)-4-(4-chlorophenyl)-furan, 1-(4-fluorophenyl)-2-(4-pyridyl)-4-(4-chlorophenyl)-pyrrole, 1-( 1-methyl-4-hydroxy-4-yl)-piperidyl-3-(4-fluorophenyl)-4-(4-pyridyl)-oxazole and in particular the di-substituted isoxazoles 3-(4-fluorophenyl)-4-(4-pyridyl)-isoxazole, 3-(4-pyridyl)-4-(4-fluorophenyl)-isoxazole, 1-(4-fluorophenyl)-2-(4-pyridyl)-isoxazole, 1-(4-fluorophenyl)-2-(4-[3-halo-pyridyl])-isoxazole and 1-(4-fluorophenyl)-2-(4-[3-alkoxy-pyridyl])-isoxazole, as well as their derivatives characterized by the presence of a methyl or ethyl group in position 3 of the isoxazole ring.

Basically, any pharmacologically acceptable p38MAPK inhibitor may be used in the treatment of inflammatory disease.

As used herein, a "tissue sample" is any portion of tissue taken from the subject for diagnostic purposes (biopsy), but preferably a sample of synovial tissue. Preferably, the size of the sample is chosen so as to allow for both molecular analysis and immunohistochemistry while at the same time not causing undue discomfort to the subject. More preferably, the sampling is performed using endoscopy. Suitable procedures are known to those skilled in the art.

As used herein, a "synovial tissue" is a soft tissue which lines the non-cartilaginous surfaces within joints with cavities, including all structural elements of this lining (synovial membrane, vasculature, etc.).

As used herein, a "benefit" is any amelioration in relevant clinical parameters or decrease in subjective suffering of the subject amenable to scoring, as well as any retardation in the progress of the disease in comparison to an untreated control, that can be causally connected to a therapeutic measure.

A "potential benefit" is thus any such benefit that may be expected to be achieved, with a reasonable chance of success, by the intended treatment under the conditions as determined, for an individual subject. In particular, as

used herein, a benefit or potential benefit may refer to any extension or reasonably expectable extension, respectively, of life expectancy and/or increase or reasonably expectable increase in the quality-adjusted life years or disability-adjusted life years (QALYs and DALYs).

As used herein, the term "level of expression" is used to denote the total amount of a certain gene product, e. g. an mRNA or, preferably, a protein, which is found in a cell, tissue, tissue sample or other biological structure at the time of measurement, standardized relative to a suitable quantity, e. g. the amount of a gene product known to be constitutively and constantly expressed in the tissue of interest, preferably of a housekeeping gene product, e. g. mRNA encoding $\alpha$-actin or, preferably, $\alpha$-actin protein, in the biological structure, or, more preferably, relative to the total content of molecules of the same class (e. g. total cellular RNA, total cellular mRNA or, preferably, total cellular protein) as the gene product of interest in the biological structure.

As used herein, the term "level of phosphorylation" is used to denote the amount of the phosphorylated form of a protein as defined before, e. g. relative to the total protein content of the cell.

The concept of "quality-adjusted life years" and "disability-adjusted life years" is used extensively to evaluate drugs and treatments, in particular in the context of those diseases where morbidity and disability are more of a concern than mortality. Essentially, each year the life time following treatment is multiplied with an index factor which ranges from 1.0 to indicate perfect quality of life, or zero disability, to 0.0 to indicate death, or complete disability, and the sum of these products is compared to the value obtainable without treatment. Suitable definitions and methods for determining gains and losses in QALYs and DALYs have been described in the art.

As used herein, "inflammatory disease" is a pathological state dominated by a localized (including polytopic) inflammation reaction and concurrent tissue damage in the absence of an obvious target or causative agent.

In a preferred embodiment of the invention, the inflammatory disease is rheumatoid arthritis.

Rheumatoid arthritis is a chronic, multisystem manifestation of inflammatory disease that commonly affects the joints in a polyarticular manner (hence also referred to as polyarthritis). The symptoms that distinguish rheumatoid arthritis from other forms of arthritis are inflammation and soft-tissue swelling of many joints at the same time. The joints are generally affected in a symmetrical fashion. The pain generally improves with use of the affected joints, and there is usually stiffness of all joints in the morning that lasts over 1 hour. Thus, the pain of rheumatoid arthritis is usually worse in the morning compared to the classic pain of osteoarthritis where the pain worsens over the day as the joints are used.

In longstanding arthritis the inflammatory activity leads to erosion and destruction of the joint surface, which impairs their range of movement and leads to deformity. This process is due not only to the side-effects of aggressive compounds and to mechanical abrasion caused by swelling, but also to active secretion of metalloproteinases and disintegrin by macrophages and fibroblasts in the inflamed area, which are capable of degrading cartilage components including type II collagen and aggrecan. Stimulation of osteoclasts is also assumed to be involved. Without wishing to be bound by theory, it appears that at least some of these corrosive functions are actually intended to serve to cleave through infested tissue to reach the pathogens.

The ensuing clinical symptoms of rheumatoid arthritis are typical: The fingers are typically deviated towards the little finger (ulnar deviation) and can assume unnatural shapes. Classical deformities in rheumatoid arthritis are the Boutonniere deformity (hyperflexion at the proximal interphalangeal joint with hyperextension at the distal interphalangeal joint) and the swan neck deformity (hyperextension at the proximal interphalangeal joint, hyperflexion at the distal interphalangeal joint). The thumb may develop a "Z-Thumb" deformity with fixed flexion and subluxation at the metacarpophalangeal joint, leading to a "squared" appearance in the hand. Subcutaneous nodules on extensor surfaces, such as the elbows, are often present. Extra-articular manifestations also distinguish this disease from osteoarthritis; hence it is a multisystemic disease. Anaemia, lung fibrosis, vasculitic disorders, renal amyloidosis, pericarditis and splenomegaly may also occur.

Thus, life expectancy for patients with untreated rheumatoid arthritis is shortened by 5 - 10 years; particularly poor prognostic factors include persistent synovitis, early erosive disease, extra-articular findings (including subcutaneous rheumatoid nodules), positive serum RF (rheumatoid arthritis factor) findings, presence of serum anti-CCP autoantibodies, carriership of HLA-DR4 "Shared Epitope" alleles, family history of rheumatoid arthritis, poor functional status, socioeconomic factors, elevated acute phase response (measured in terms of e. g. erythrocyte sedimentation rate and C-reactive protein), and increased clinical severity.

Potentially disabling morbidity, however, is considerably greater: After 5 years of disease, approximately 33% of patients will not be working; after 10 years, approximately half will have substantial functional disability. The skilled artisan will be aware of methods for the evaluation of pain and disability (scoring scales) and will also appreciate the high influence of psychological factors (strong placebo effect).

Rheumatoid arthritis occurs most frequently in the 20 - 40 age group (incidence is estimated to be about 0.3 % to 1.0 % in different ethnic groups), although it can start at any age. It is strongly associated with the HLA marker DR4, hence family history is an important risk factor. The disease affects females : males in a 3 : 1 ratio.

In 1987, the American College of Rheumatology has defined the following criteria for the diagnosis of rheumatoid

arthritis:

- Morning stiffness of more than 1 hour;
- arthritis and soft-tissue swelling of more than three of 14 joints/joint groups;
- arthritis of hand joints;
- symmetric arthritis;
- presence of subcutaneous nodules in specific places;
- rheumatoid factor at a level above the 95th percentile; and
- radiological changes suggestive of joint erosion. At least four of these criteria have to be met to establish the diagnosis, although currently many patients are treated "at suspicion" despite not or not fully meeting the criteria.

In a further preferred embodiment of the invention, the inflammatory tissue is synovial tissue, in particular synovial tissue from the finger and toe joints (MCP = metacarpophalangeal joint, MTP = metatarsophalangeal joint, PIP = proximal interphalangeal joint) and knee joints, and it is particularly preferred that the synovial tissue sample from the subject is obtained by synovectomy.

As used herein, a "synovectomy" is a surgical procedure whereby a sample of synovial tissue is obtained from a subject, preferably a living subject, more preferably a living mammal subject, e. g. a living human, suspected or diagnosed of having inflammatory disease. Suitable procedures are known to those skilled in the art. For an overview see Connor J & Nalebuff EA, Curr. Opin. Rheumatol. 7(1995) : 120-124.

In a further preferred embodiment of the invention, determining p38MAPK expression comprises detecting p38MAPK protein, preferably by using an antibody against p38MAPK and more preferably by using said antibody in a method which is selected from the group consisting of immunoblotting (Western blotting), immunoprecipitation and immunohistochemistry. These methods have been described in the art.

As used herein, an "antibody" is a protein or glycoprotein molecule comprising a specific target binding domain derived from the variable regions of an immunoglobulin molecule, preferably a protein or glycoprotein molecule selected from polyclonal antibodies, e. g. polyclonal antisera; monoclonal antibodies, e. g. antibodies obtained from hybridomas; recombinant antibodies, e. g. antibody fusion proteins, chimeric and humanized antibodies; and fragments of polyclonal, monoclonal or recombinant antibodies, e. g. Fab and F(ab)2 fragments of the same. More preferably it is a monoclonal antibody, most preferably a monoclonal antibody of type IgG, e. g. a murine monoclonal IgG antibody. The antibody may carry a covalent chemical modification, preferably such as facilitates detection of the antibody, e. g. a fluorescent label.

Antibodies may be obtained by a variety of methods, all of which are in accordance with the present invention. Basically, any antibody with suitable binding characteristics may be used in the method of the invention.

A polyclonal antiserum, herein understood to comprise one or more antibodies against the same target molecule but with different binding regions and potentially also with different binding epitopes on said target molecules, may be obtained by immunizing a vertebrate, preferably a mammal, more preferably a mammal selected from the group consisting of sheep, goat, horse, donkey, rabbit, rat and mouse, with the antigen or a conjugate of the antigen, e. g. the antigen covalently coupled to keyhole limpet haemocyanin (KLH), in the presence of a suitable adjuvant, e. g. Freund's adjuvant. Suitable procedures and formulations for immunization have been described extensively in the art. Following bleeding of the animals and removal of the particulate blood components such as blood cells by a suitable method, e. g. centrifugation, the antiserum can be obtained by a method such as affinity chromatography, e. g. affinity chromatography over columns wherein a molecule with specific affinity for immunoglobulin, preferably staphylococcal Protein A or Protein G, has been immobilized on the matrix. Such methods are well-known to those skilled in the art, as are methods for determining purity, e. g. electrophoresis, and affinity, e. g. sorbent assays such as RIA or ELISA.

As used herein, the term "monoclonal antibody" means an antibody obtainable or derived from a hybridoma, e.g. an antibody secreted by a hybridoma prepared by means of hybridoma technology such as the standardized hybridoma methods according to Kohler and Milstein or any modified method based thereupon. Essentially, the standard method comprises immunizing an animal, preferably a rodent and more preferably a mouse, with the antigen as described above, followed by isolating the immunized animal's spleen cells, fusing them with an immortal cell line and assessing the cell fusion products for antibody production. An antibody which is derived from a hybridoma and which has specificity for a target molecule of the invention, preferably for p38MAPK and more preferably for p38MAPK$\alpha$, or derivative thereof is therefore referred to as a monoclonal antibody.

As used herein, the term "recombinant antibody" refers to antibodies which are produced, expressed, generated or isolated by recombinant means, such as antibodies which are expressed using a recombinant expression vector transfected into a host cell; antibodies isolated from a recombinant combinatorial antibody library; antibodies isolated from an animal (e.g. a mouse) which is transgenic due to human immunoglobulin genes (see, for example, Taylor, L.D., et al. (1992) Nucl. Acids Res. 20:6287-6295); or antibodies which are produced, expressed, generated or isolated in any other way in which particular immunoglobulin gene sequences (such as human immunoglobulin gene sequences)

are assembled with other DNA sequences. Recombinant antibodies include, for example, chimeric, CDR graft and humanized antibodies.

An antibody or antibody moiety of the invention may generally be produced by recombinantly expressing the genes for light and heavy immunoglobulin chains in a host cell. In order to recombinantly express an antibody, a host cell is transfected with one or more recombinant expression vectors carrying DNA fragments encoding the light and heavy immunoglobulin chains of said antibody, thereby expressing the light and heavy chains in the host cell and secreting them preferably into the medium in which said host cells are cultured. The antibodies can be isolated from this medium. Standardized recombinant DNA methods are used in order to obtain genes for heavy and light antibody chains, e. g. by deriving them by RT-PCR from the genes expressed in a particular hybridoma cell, to insert said genes into recombinant expression vectors and to introduce said vectors into host cells. Methods of this kind are described, for example, in Sambrook, Fritsch and Maniatis (eds.), Molecular Cloning; A Laboratory Manual, Second Edition, Cold Spring Harbor, N.Y., (1989) (hereinafter referred to as "Sambrook"), Ausubel, F.M. et al. (eds.) Current Protocols in Molecular Biology, Greene Publishing Associates, (1989) and in US 4,816,397 by Boss et al..

Standard recombinant DNA and molecular cloning techniques used herein are well-known in the art and are also described more fully in Sambrook.

As used herein, the general term "recombinant" refers to an artificial combination of at least two otherwise separated segments of sequence, e.g., by chemical synthesis or by the manipulation of isolated segments of nucleic acids by genetic engineering techniques. In particular, it refers to a method of expression wherein a nucleic acid sequence encoding the protein of interest, which in itself may be either native or recombinant, is operably linked to functional sequences such as promoters, enhancers and/or terminators for the purpose of expression.

As used herein, a "specific antibody" is an antibody which, under the conditions of use, binds to the target named but essentially does not bind to other molecules; thus, a p38MAPK-specific antibody is an antibody which binds to p38MAPK but essentially not to non-p38MAPK molecules.

As used herein, an "antibody against p38MAPK" is an antibody whose specificity for p38MAPK is sufficiently high under the conditions of use to enable its use in a method according to the invention.

It is particularly preferred that the antibody used is an antibody against one of the four isoforms of p38MAPK, more preferred that it is against p38MAPK$\alpha$, and most preferred that it is specific for p38MAPK$\alpha$.

Determination of p38MAPK expression in the tissue sample may essentially be performed in situ or in vitro, or by any combination of these two possibilities applied to different portions of the sample.

For in vitro determination of p38MAPK expression, the sample is dissolved mechanically and/or chemically; in the latter case, dissolution may be performed in a suitable way to obtain the protein, in particular the p38MAPK protein, in either natural (native) or denatured form, "denatured" herein being used to refer to a partial or total loss of secondary and/or tertiary structure of the protein, preferably accompanied by partial or total cloaking of the natural electric charge of the molecule by uniform non-covalent attachment of strongly charged small molecules, e. g. by boiling with detergents, e. g. sodium dodecyl sulfate (SDS), under reducing conditions, e. g. in the presence of $\beta$-mercaptoethanol. The antibody of the invention may be able to selectively bind to the p38MAPK protein in its native form, in its denatured form, or both.

Expediently, after dissolution the total proteins are separated according to their physical properties. For denatured proteins, separation is preferably performed by size, using electrophoresis on an SDS-polyacrylamide gel; for native proteins, both by size and isoelectric point, using two-dimensional isoelectric focussing on a pH gradient gel. These techniques are familiar to the person skilled in the art.

Subsequently, the separated proteins are subjected to detection using an antibody of the invention as primary antibody. The antibody against the p38MAPK protein may be directly labelled with a quantifiable tag, e. g. a fluorescent label, e. g. FITC or PE, or an enzymatically active group, e. g. peroxidase, or, preferably, it may be unlabelled. In the latter case, detection requires the use of a secondary antibody which is directed against the primary antibody and possesses a quantifiable tag as described above. Such detectable groups and methods for determining them are known in the art.

In both cases, antibody binding is followed by a suitable detection procedure, which may employ the fluorescent properties of the label or the enzymatic activities, e. g. by providing a chromogenic substrate whose conversion into a dye is then measured photometrically.

The use of defined amounts of purified p38MAPK protein, e. g. recombinantly expressed p38MAPK protein, as standards allows for conversion of the physical data (fluorescence intensity, optical density, etc.) into defined amounts of p38MAPK protein, which may then be standardized relative to the total protein content in the tissue sample by determining the total protein content in the latter, e. g. by a colorimetric method, e. g. Bradford's protein assay. Alternatively, and more expediently for all-immunology methods such as protein arrays, the p38MAPK content may be standardized relative to the amount of a protein known to be constitutively and constantly expressed in the tissue of interest, preferably a housekeeping protein, e. g. $\alpha$-actin. The amount of this reference protein may be determined by methods analogous to those for p38MAPK. Suitable reference proteins have been described in the art.

Alternatively, for in vitro determination proteins may be selectively precipitated from the dissolved sample using

suitable antibodies, e. g. antibodies covalently linked to insoluble matrix materials, e. g. latex or polystyrol beads, and quantified directly, then standardized relative to the total protein content in the tissue sample as described above, or, preferably, be subjected to a step of separation as described above in order to determine covalent modifications, preferably phosphorylations and more preferable tyrosine phosphorylations which are the hallmark of p38MAPK activation, thereby allowing to determining the percentage of p38MAPK protein which is in the activated state.

In a particularly preferred embodiment of the invention, immunoprecipitation is followed by electrophoretic separation and detection with a second antibody, e. g. an anti-phosphotyrosine antibody, more preferably an anti-phosphotyrosine antibody which is specific for phosphotyrosine in the natural peptide context of the p38MAPK phosphorylation site, in particular specific for p38MAPK over other protein kinases, e. g. specific for a peptide comprising a coherent run of 1, 2 or 5 amino acid residues of the p38MAPK sequence adjacent on each side of the phosphorylable TGY motif.

In another particularly preferred embodiment of the invention, immunoprecipitation is performed using a method selected from enzyme-linked immunosorbent assay (ELISA) and radioimmunoassay (RIA), preferably using a sandwich ELISA.

It is especially preferred that the detection method employs two antibodies against distinct epitopes of p38MAPK, which do not sterically or otherwise interfere with each other's binding; one of these is attached to a substrate, preferably covalently linked to a solid matrix, and the other to a group for detection, e. g. a fluorescent or enzymatically active group. The detection method comprises bringing the sample into contact with the first antibody (hereinafter referred to as "capture antibody") attached to a substrate, washing out impurities showing low-affinity binding to the substrate, adding the labelled second antibody (hereinafter referred to as "detection antibody") and detecting the fluorescent or enzymatically active group by a suitable method, e. g. directly by fluorometry or colorimetrically by addition of a chromogenic substrate which is then converted into a dye by the enzymatic activity of the label, followed by determination of optical density at a predefined wavelength. Alternatively, the detection antibody may be labelled with biotin and the detectable group coupled with avidin or streptavidin, thereby making it possible for more detectable groups to cluster around a single detection antibody molecule, thus achieving a stronger amplificatory effect and hence a more advantageous signal : noise ratio.

In a most preferred embodiment of the invention, an antibody specific for p38MAPK, preferably specific for p38MAPK$\alpha$, is immobilized, by adsorption or by covalent attachment, to the inner surface of a plastic reagent vessel, preferably of a 96-well microtiter plate, so to serve as capture antibody; a tissue lysate obtained from a representative portion of tissue, preferably comprising about 1 x $10^7$ cells, is solubilized in a suitable lysis buffer, preferably an approximately neutral, e. g. pH 7 - 7.5, lysis buffer comprising adequate amounts of chelators, detergents and protease inhibitors to avoid degradation of the protein(s) of interest, e. g. EDTA, Triton X-100, sodium fluoride, urea and peptidic protease inhibitors, is brought into contact with the immobilized capture antibody; unbound protein and impurities are washed away, preferably using a balanced salt solution, more preferably phosphate-buffered saline comprising a mild detergent; a biotin-conjugated antibody selected from the group consisting of an antibody specific for p38MAPK, preferably specific for p38MAPK$\alpha$, whose binding is not interfered with by that of the capture antibody, and an antibody specific for phosphorylation, preferably tyrosine phosphorylation, more preferably for phosphorylation of Tyr-182, is applied, followed by a second round of washing as described; an streptavidin-horseradish peroxidase conjugate is added, followed by a third round of washing as described; a substrate solution comprising a chromogenic substrate for horseradish peroxidase, preferably a solution comprising $H_2O_2$ and tetramethylbenzidine, is applied and incubated for a predetermined period of time prior to termination of the reaction by addition of acid, preferably concentrated sulfuric acid; optical density is measured, preferably at $\lambda$ = 450 nm; and the amount of p38MAPK or phosphorylated p38MAPK is determined by comparison with internal standards with defined amounts of the same, e. g. recombinantly expressed and purified.

In another particularly preferred embodiment of the invention, the ELISA is implemented as a protein array or microarray as described, e. g., by Balboni et al. 2006 (Annu. Rev. Immunol. 2006, Apr 23, 24 : 391 - 418); MacBeath et al. 2000 (Science 289(5485), 1760 - 1763). Detection of proteins bound to arrays or mi-croarrays may be done by using fluorescent labels attached to the proteins of interest or to secondary antibodies, or by surface plasmon resonance as described by Huang and Chen 2006 (Biosens. Bioelectron. 2006 Mar 8), or other methods.

In a further particularly preferred embodiment of the invention, quantification of p38MAPK in synovial tissue samples is performed using a ready-made kit analogous to the one described by Katus et al. for the quick determination of cardiac troponin in cases of suspected heart infarction (Katus et al. 1992, Clin. Chem. 1992 Mar; (3): 386 - 393; marketed by Boehringer Mannheim under the name of "Enzymun-Test® Troponin T"). Preferably, the kit implements parallel quantification of p38MAPK and a reference protein as described above, and more preferably, parallel quantification of p38MAPK, phosphorylated p38MAPK and a reference protein, e. g. $\alpha$-actin.

All of the aforementioned in vitro methods will finally yield the,total p38MAPK content (w/w) of the tissue sample and, optionally, more detailed information about the biochemical status of the protein of interest, e. g. the degree of p38MAPK activation by phosphorylation. These values are then used as part of the matrix or profile E to estimate, by correlation with previous clinical experience embodied in a function F as described in formula (1) and (2) above, the

subject's potential benefits.

As used herein, "in situ" refers to any method of detection wherein the sample is not dissolved but observed in its natural structure, thereby enabling the skilled artisan to obtain information about the spatial distribution of the protein of interest. Preferably, in situ detection is performed using sections, e. g. microtomic sections, of the tissue sample, expediently fixed and/or embedded. Suitable methods for sample preparation are well-known to those skilled in the art. In a preferred embodiment of the invention, the sample is fixated, e. g. by treatment with glutardialdehyde, embedded into a suitable solidifying medium, e. g. microscopy grade high molecular weight polyvinyl alcohol (e. g. Mowiol™), and cut into a series of sections using a microtome, preferably sections with a thickness ranging from 1 to 50 $\mu$m and more preferably from 3 to 10 $\mu$m, which are then mounted upon a suitable support, e. g. a microscope slide, and subjected to immunohistological staining.

As used herein, "immunohistological staining" is any method that allows for specific detection of distinct molecule species in a tissue sample for optical, preferably for microscopic analysis in situ. In a preferred embodiment of the invention, immunohistological staining is performed by soaking a microscopy sample, prepared as described above, into a suitably diluted solution of a primary antibody against the structure of interest, then washing it to remove excess antibody, adding a secondary antibody which is directed against the first antibody and conjugated with a fluorescent group, washing out excess antibody once more and subjecting the stained sample to fluorescence microscopy as appropriate. By using primary antibodies derived from different species and suitable secondary antibodies with distinct labels, more than one target can be assessed in parallel, and colocalization of different structures can be determined, e. g. the presence of a protein and a common activation marker such as phosphorylation. Materials and procedures applicable to this task are known to those skilled in the art. It is particularly preferable that analysis of the micro-scopic images is performed using automatic evaluation software which allows for suitable quantifications of the micrographs. The results thus obtained, e. g. a two-dimensional matrix giving a map of the distribution of expression and phosphorylation values within a cell or tissue, are useful as components of the expression matrix or profile E in formula (1) or (2) as described above, once their clinical significance has been quantified.

Preferably, both approaches are combined to obtain a complete set of p38MAPK expression data, with a part of the sample being dissolved for in vitro determination and the rest for in situ measurements.

In a further preferred embodiment of the invention, the antibody against p38MAPK does not discriminate between activated (tyrosine-phosphorylated) and resting-stage p38MAPK, but a distinct antibody may be used to detect activation, e. g. an anti-phosphotyrosine antibody, so that both characteristics of the activated enzyme may be determined, either by pre-selection prior to detection (e. g. immunoprecipitation of p38MAPK followed by phosphotyrosine detection, or immunoprecipitation of tyrosine-phosphorylated proteins followed by p38MAPK detection), or by dual-channel measuring with ensuing correlation of the values thus obtained (e. g. using two antibodies or primary/secondary antibody couples with different specificities and fluorescent labels and comparing fluorescence intensities). Suitable methods are well-known to those skilled in the art.

In a further preferred embodiment of the invention, determining p38MAPK expression comprises detecting p38MAPK mRNA, preferably by using a method which is selected from the group consisting of hybridization (Northern blotting) and RT-PCR. These methods have been described in the art.

In a particular embodiment of the invention, the detection of p38MAPK mRNA is quantitative. More particularly, the p38MAPK mRNA is quantified in relation to the expression of other protein kinases, e. g. such as are capable of phosphorylating p38MAPK ("upstream kinases").

In a particular embodiment of the invention, determining p38MAPK expression comprises determination of the sequence of the full length or parts of the p38MAPK mRNA and/or of the genomic DNA encoding the same, including the nucleotide sequences controlling p38MAPK expression, e. g. promoters, enhancers, terminators and splice sites. As used herein, the term "determination of nucleotide sequences" is meant to comprise sequence analysis, e. g. by the didesoxynucleotide chain termination method, optionally in combination with RT/PCR, as well as determination of the presence or absence of a particular pre-defined sequence tag by any method comprising a hybridization step. Such methods are well-known to the person skilled in the art.

In a more particular embodiment of the invention, the determination of a nucleotide sequence of p38MAPK expressed in the sample is used for the identification of aberrations, variants and/or polymorphisms, in particular such as may account for either particularly high or particularly low levels of p38MAPK activity and/or for an increased probability of either particularly high or particularly low levels of p38MAPK activity, and/or as may be related to the binding of an inhibitor to the p38MAPK protein present in the inflammatory sample and/or other factors capable of influencing p38MAPK activity.

It is particularly preferred in the method of the present invention that the p38MAPK is p38MAPK$\alpha$. It is also particularly preferred that the p38MAPK$\alpha$ comprises non-phosphorylated p38MAPK$\alpha$ and phosphorylated p38MAPK$\alpha$. In a particular embodiment, the p38MAPK$\alpha$ is total p38MAPK$\alpha$.

It is particularly preferred in the method of the present invention that the expression of the p38MAPK is expression in the synovial lining.

It is also particularly preferred in the method of the present invention that the expression of the p38MAPK is expression on macrophages.

Preliminary data suggest that the high-p38MAPKα phenotype may be linked to rather recent onset (< 5 years) of inflammatory disease, whereas very long standing of inflammatory disease (> 20 years) was predominantly found associated with the low-p38MAPK phenotype. This may suggest a potential two-step progression of the disease, where at least in some patients p38MAPK is particularly involved into the generation of a state of chronic inflammation, whereas following the establishment of the disease other mechanisms take over. In this case, classification of inflammatory disease as being either high-p38MAPK, corresponding to an earlier stage of the disease, or low-p38MAPK, corresponding to fully established disease, might even influence the selection of suitable further therapeutic measures beyond the scope of p38MAPK inhibitor administration. In particular, the established stage with low-p38MAPK phenotype (and consequently low prospects of benefit to be expected from the administration of p38MAPK inhibitors) may be taken to implicitly recommend other therapeutic approaches, which will gain importance as the availability of molecular medicine tools increases.

For the time being, this classification may influence decisions regarding more radical measures such as surgical joint replacement, which may be more appropriate in patients which cannot be expected to substantially benefit from p38MAPK inhibitor administration, or in which the disease has generally reached a state of establishment where it is no longer easily accessible to pharmacotherapy (i. e. low-p38MAPK or established disease), whereas in high-p38MAPK or early disease more conservative treatment regimes might be advisable.

Thus, in a further aspect the present invention relates to a method of classifying the clinically relevant stage of inflammatory disease in a subject having the same, which comprises

(I) obtaining an inflammatory tissue sample from the subject; and
(II) determining whether p38MAPK is expressed in the tissue sample,
the expression of p38MAPK indicating whether the disease is in its early or in its established stage.

Herein, steps (I) and (II) are performed as described above for the estimation of potential benefits from administration of p38MAPK inhibitors.

In another aspect, the present invention relates to the use of an antibody against the p38MAPK protein for determining whether a subject having or suspected of having inflammatory disease may benefit from treatment with a p38 mitogen-activated protein kinase (p38MAPK) inhibitor. The amount of protein that is bound by the antibody against p38MAPK indicates the subject's potential benefits, and administration of p38MAPK inhibitors can be restricted to the p38MAPK-expressing subgroup of patients where it will be effective. Preferably, the antibody is specific for p38MAPKα.

In a further aspect, the present invention relates to the use of a nucleic acid sequence essentially complementary or identical to the full length or to a part of the sequence encoding p38MAPK, wherein the term "essentially" is meant to denote a degree of complementarity or identity that is sufficient to allow hybridization under the conditions of the experiment, for determining whether a subject having or suspected of having inflammatory disease may benefit from treatment with a p38MAPK inhibitor. In a particular embodiment, the nucleic acid sequence is labelled with a group facilitating detection, e. g. with a fluorescent or radioactive group, a moiety possessing enzymatic activity or a hapten, so it is useful in the detection and/or quantification of p38MAPK mRNA and/or particular aberrations, variants and/or polymorphisms within the same.

Preferably, the amount of labelled nucleic acid that is bound by the p38MAPK mRNA present in the sample indicates the subject's potential benefits, and administration of p38MAPK inhibitors can be restricted to the p38MAPK-expressing subgroup of patients where it will be effective. More particularly, the nucleic acid may be used to identify sequences within the p38MAPK sequence having an impact on the efficacy of a p38MAPK inhibitor.

Methods for assessing the specific binding of a labelled nucleotide to a target within a population of sequences are well-known to the skilled artisan and comprise enzymatic methods, e. g. PCR and RT/PCR, as well as non-enzymatic methods, e. g. Northern and Southern blotting and the methods derived therefrom.

It is particularly preferred that the nucleotide sequence is complementary or identical to the full length or to a part of the sequence encoding p38MAPKα.

In a further aspect, the present invention relates to a kit for determining from an inflammatory tissue sample, preferably an inflammatory tissue sample obtained by synovectomy, whether a subject having or suspected of having inflammatory disease may benefit from treatment with a p38 mitogen-activated protein kinase (p38MAPK) inhibitor. This kit comprises an antibody against p38MAPK, preferably an antibody specific for p38MAPKα, and an instruction for the use of said antibody to determine expression of the p38MAPK and thereby estimate the subject's potential benefits, as described above.

Preferably, this kit also comprises a means for standardizing the amount of p38MAPK, more preferably a means for quantifying the total protein in the sample and/or quantifying a protein with constitutive expression in the tissue of interest. More preferably, it comprises an antibody against a-actin and instructions for the use of said antibody to

determine a-actin expression. It is most preferred that the reagents and methods required for determination of p38MAPK and the reference protein are formulated in such a way that they allow for parallel determination of the protein of interest and the reference protein in a single pass of operation.

As used herein, "constitutive expression" is understood to be the expression of a gene, in particular a gene encoding a protein, that shows little, preferably no detectable, difference in expression between cells and/or tissues subjected to different stimuli and/or conditions of growth, in particular little or preferably no difference in expression between inflamed and non-inflamed tissue of the same type.

In yet a further aspect, the present invention relates to use of a kit for determining p38MAPK expression for estimating whether a subject having or suspected of having inflammatory disease may benefit from treatment with a p38 mitogen-activated protein kinase (p38MAPK) inhibitor, using formula (1) or (2) to determine the probability of beneficial effects B from the expression profile and other characteristics of the individual subject, as described above.

Preferably, this comprises the use of a correlative function or table of any kind giving the likelihood of benefits to be expected from treatment with p38MAPK inhibitors, preferably with specific p38MAPK$\alpha$ inhibitors, for a subject with the specific level of p38MAPK expression determined using the kit, as can be estimated from previous clinical trials. In a particularly preferred embodiment of the invention, such a correlative function or table is provided as a component of the kit.

The present invention will now be described in more detail with reference to the following examples and drawings which illustrate the invention without limiting it in any respect.

In the figures,

Fig. 1 (A) shows immunoblots of recombinant p38MAPK$\alpha$, p38MAPK$\beta$, p38MAPK$\gamma$ and p38MAPK$\delta$, each stained using primary antibodies against the respective isoform and appropriate secondary antibodies conjugated with horseradish peroxidase; and
(B) shows a panel of four immunoblots of three pools of synovial tissue from patients (200 $\mu$g total protein per sample) with rheumatoid arthritis stained with distinct antibodies against each of the four isoforms after SDS-PAGE separation and transfer of the proteins to a nitrocellulose membrane.

Fig. 2 (A) shows immunoblots of synovial tissue from 14 different patients with rheumatoid arthritis (200 $\mu$g total protein per sample), each probed with an antibody against one of p38MAPK$\alpha$, p38MAPK$\beta$, p38MAPK$\gamma$, p38MAPK$\delta$ and phosphotyrosine, respectively, and with a-actin as a positive control, and
(B) is a diagram showing the relative number of patients with strong (left column of each diagram), weak (middle column) and undetectable (right column) expression of the respective p38MAPK isoform.

Fig. 3 (A) shows the immunohistochemistry of p38MAPK$\alpha$, p38MAPK$\beta$, p38MAPK$\gamma$ and p38MAPK$\delta$ in the synovial layer (left column) and the synovial microvasculature (right column) of patients with rheumatoid arthritis. Brown colour indicates positive staining, and
(B) quantification of the expression of p38MAPK in the following different synovial subcompartments: lining layer, sublining, vessel wall and vascular endothelium. For each p38MAPK isoform in each subcompartment, the number of positive cells is given.

## EXAMPLES

### Example 1 - Isoform specificity of anti-p38MAPK antibodies

Since the assessment of p38MAPK isoforms depends on the specificity of antibodies against the various isoforms of p38MAPK, which do not show cross reactivity with other p38MAPK isoforms, it was necessary to first ascertain isoform specificity of the antibodies selected for the study, namely:

- rabbit polyclonal anti-p38MAPK$\alpha$ ;
- mouse monoclonal anti-p38MAPK$\beta$ (clone 11A5) ;
- mouse monoclonal anti-p38MAPK$\gamma$ (clone 212464) ; and
- rabbit polyclonal anti-p38MAPK$\delta$.
  While Western blotting recombinant p38MAPK isoforms and probing them with antibodies against each of these isoforms, a monospecific binding was observed. This suggests that there is no relevant cross-reactivity among the isoform present (Figure 1A).

To confirm this result obtained with recombinant proteins and to extend the specificity of the antibodies to the situation in vivo, the inventors next probed pools (N = 3) of synovial tissue extracts from patients with rheumatoid arthritis,

(N = 14) with these antibodies specific for different p38MAPK isoforms. Once more all antibodies emerged as specific by recognizing one single band instead of multiple bands, assuming that synovial tissue is a source of more than one p38MAPK isoform (Figure 1 B).

These findings demonstrate that the antibodies used are suitable tools for discriminating between the individual p38MAPK isoforms, and in particular for assessing the relative up- or downregulation of any of these relative to the expression level in a predetermined reference tissue, which will be generally a noninflammatory tissue of the same type.

Example 2 - Clinical study

2.1. Patient characteristics

The inventors of the present invention analysed expression in differential activation of p38MAPK isoforms in synovial tissue from 14 patients with rheumatoid arthritis. The characteristics of these patients are given in table 1. Of these 14 patients, 12 were female and 2 were male. On average, their age was 48,6 $\pm$ 13,3 years. These patients had had an average duration of disease of 11,5 $\pm$ 8,9 years, indicating a long-standing rheumatoid arthritis.

The acute phase reactants were fairly high with a mean of C-reactive protein (CRP of 6,16 $\pm$ 3,27 mg/dl) and an erythrocyte sedimentation rate of 40,5 $\pm$ 25,79 mm/h. Of these 14 patients, 11 had erosive disease as determined by plain X-rays of hands and feet. With respect to therapy, several DMARDs were used: Seven of the 14 were on methotrexate, five of the 14 were on leflunomide (of these, three also received methotrexate) and each one of the 14 on salazopyrine and on tumour necrosis factor blocker (etanercept; additionally also received methotrexate), respectively. One patient was off the DMARDs and received glucocorticoids only, which were additionally given in almost all patients (13 of the 14; the only exception being one patient who was on methotrexate only).

2.2. Immunoblotting reveals predominant p38MAPK$\alpha$ and p38MAPK$\gamma$ expression in the synovial tissue of patients with rheumatoid arthritis

Synovial tissue extracts from the 14 patients included into the study were assessed for expression of the four distinct p38MAPK isoforms (Fig. 2A). It was found that p38MAPK$\alpha$ and p38MAPK$\gamma$ are the predominant isoforms expressed in the synovial tissue of rheumatoid arthritis patients, with each of these being strongly expressed in 57 % of the patients, whereas strong expression of p38MAPK$\beta$ and p38MAPK$\delta$ was found only in 7 % and 14 % of the patients, respectively.

2.3. Expression of p38MAPK$\alpha$ and p38MAPK$\gamma$ but not of p38MAPK$\beta$ and p38MAPK$\delta$ is linked to increased levels of C-reactive protein

Levels of C-reactive protein were found to be higher, on average, in patients with high levels of p38MAPK$\alpha$ and p38MAPK$\gamma$ in the synovial membrane than those with lover levels, whereas there was no significant difference in C-reative protein levels observed between patients with high and those with low levels of p38MAPK$\beta$ and p38MAPK$\delta$.

These findings argue for the causal participation of p38MAPK$\alpha$ and p38MAPK$\gamma$, where they are upregulated, but not of p38MAPK$\beta$ and p38MAPK$\delta$, in an inflammatory mechanism also involving the upregulation of C-reactive protein. As discussed above, C-reactive protein has long been known to be upregulated only in a subset of rheumatoid arthritis patients, but this upregulation is considered as prognostically infaust where it occurs, which corroborates the assumption that the molecular mechanisms underlying high-p38MAPK and low-p38MAPK inflammatory disease are markedly different, necessitating differential treatment.

2.4. The synovial lining and microvasculature are the primary expression sites of p38MAPK isoforms and activated p38MAPK

Differential expression of p38MAPK isoform expression within the synovium was characterized by in vivo staining (figure 5), and the following subcompartments were identified: Strong expression of all four isoforms in the synovial lining; weak expression of all four isoforms in the synovial sub-lining; marked p38MAPK$\delta$ expression in the synovial microvessel wall; and marked p38MAPK$\gamma$ expression in the microvessel endothelium.

These findings further argue for specific roles of the different p38MAPK isoforms in rheumatoid arthritis.

2.5. Dual labelling with cell specific markers reveals macrophages and synovial fibroblasts as foci of p38MAPK expression

By double-labelling with cell-type specific markers, in situ staining was used to attribute p38MAPK expression to different cell types. It was shown that synovial macrophages preferentially expressed p38MAPK$\alpha$ and p38MAPK$\gamma$,

whereas synovial fibroblasts mainly express p38MAPKβ and p38MAPKδ but no p38MAPKα (Table II). In other cell types, p38MAPK expression was generally low.

These findings support the notion that synovial fibroblasts and macrophages are instrumental in rheumatoid arthritis.

2.6. Predominance of the α and γ isoforms among activated p38MAPK from inflammatory tissues

Phosphorylated p38MAPK in pooled protein lysates from synovectomy samples was immunoprecipitated using anti-phosphotyrosine and characterized using the isoform-specific antibodies. It was shown that the lysates were rich in phosphorylated p38MAPKα and p38MAPKγ, whereas p38MAPKγ was scarce and p38MAPKδ below the detection threshold.

These findings support the importance of p38MAPKα and p38MAPKγ activation for rheumatoid arthritis.

2.7. Differential role of p38MAPKα in early and late stages of the disease

With a standard deviation of 77 % all in all, there does not seem to exist a clear correlation between p38MAPKα expression and disease. When, however, discarding the "fresh" cases (< 5 YOD), i. e. patients 7, 8, 1 and 9, it becomes clear that among these p38MAPKα expression is particularly high, whereas the long-time 2 and 13 are lacking not only in p38MAPKα but in p38MAPK expression altogether. This reveals that p38MAPKα is particularly important in establishing rather than maintaining the inflammatory state.

Without wishing to be bound by theory, it is assumed that the initial damage, mediated by p38MAPK and in particular by p38MAPKα, might then come into play as a feedback activator causing secondary effects which are not necessarily mediated by p38MAPK and thus induce further damage. This is considered to imply that in low-p38MAPK disease a state has been reached that cannot be easily broken pharmacologically (such a state probably involving epigenetic changes as well as the formation of novel and mutually stimulating arrangements of cells, as has been demonstrated for atherosclerosis), and that therefore suggestions of more drastic measures, e. g., surgical intervention, should be received more openly in these cases.

Example 3 - ELISA for p38MAPK or p38MAPKα

3.1. ELISA for the p38MAPK protein

An antibody against human p38MAPKα was diluted to 4.0 μg/ml in phosphate-buffered saline and used to coat a 96-well microtiter plate (Costar EIA plate, #2592) by applying 100 μl of the solution to each well. The plate was then sealed and incubated overnight at room temperature to allow the antibody to adsorb to the plastic.

Each well was then aspirated and washed thrice with wash buffer (0.05 % Tween 20 in phosphate-buffered saline, pH 7.3), using approximately 400 μl of wash buffer per well each time. After the last round of washing, residual wash buffer was removed by aspiration.

To each well, 300 μl of blocking buffer (1 % bovine serum albumin and 0.05 % $NaN_3$ in phosphate-buffered saline, pH 7.3) were added in order to saturate unspecific binding capacities, and the microplate was incubated for two hours at room temperature, followed by three further rounds of washing as described before.

During the preparation of the plate, the sample was carefully minced and washed with phosphate-buffered saline, then suspended in lysis buffer (1 mM EDTA, 0.5 % Triton X-100, 5 mM sodium fluoride, 6 M urea, 10 μg/ml leupeptin, 10 μg/ml pepstatin, 100 μM phenylmethylsulfonylfluoride, 3 μg/ml Aprotinin, 2.5 mM sodium pyrophosphate, 1 mM sodium orthovanadate in phosphate-buffered saline, pH 7.3), vortexed and kept on ice for 15 minutes to allow the tissue to dissolve. Particulates were removed prior to use by centrifugation (2000 g for 5 minutes), then the cleared lysate was used to produce a serial dilution (1:2 per each of seven steps) using dilution buffer (1 mM EDTA, 0.5 % Triton X-100, 5 mM sodium fluoride in phosphate-buffered saline, pH 7.3, optionally supplemented with urea so to adjust the final concentration of urea to 1 M before dispensing it into the coated microtiter plate).

At the same time, a serial dilution of a known amount of recombinant p38MAPK (1 : 2 per each of seven steps) was made to serve as internal standard, starting at a concentration of 20 ng/ml.

Per well of the microtiter plate, 100 μl of any of the serial dilutions were applied. The plate was then sealed hermetically and allowed to incubate at room temperature for two hours, then washed as described above and finally dried by aspiration.

Next, the biotin-conjugated capture antibody, diluted in phosphate-buffered saline, was added to the wells and incubated for two hours at room temperature. Then unbound capture antibody was removed by three rounds of washing as described above.

Then, the streptavidin-horseradish peroxidase conjugate, diluted in phosphate-buffered saline, was added to

the wells and incubated for twenty minutes at room temperature and in the dark. Then unbound conjugate was removed by three rounds of washing as described above.

For detection, 100 μl of substrate solution ($H_2O_2$ and tetramethylbenzidine in aqueous solution) were added to each well and incubated for twenty minutes at room temperature and in the dark. Then the reaction was stopped by adding 50 μl of 2 M $H_2SO_4$ to each well. A microplate reader was then used to determine optical density, net density being $OD_{450}$- $OD_{570}$, and the actual concentration of p38MAPK in the samples was calculated using the standards.

3.2. ELISA for the phosphorylated form of p38MAPK

The procedure was as described for p38MAPK, with the exception that the detection antibody was against phosphorylated tyrosine, and the recombinant protein was phosphorylated by expression in a suitable system.

## Claims

**1.** Method of determining whether a subject having or suspected of having inflammatory disease may benefit potentially from treatment with a p38 mitogen-activated protein kinase (p38MAPK) inhibitor, which comprises

(I) obtaining an inflammatory tissue sample from the subject; and
(II) determining whether p38MAPK is expressed in the tissue sample, the expression of the p38MAPK indicating the subject's potential benefits.

**2.** The method of claim 1, wherein indicating the subject's potential benefits comprises the use of a correlative function or table giving the likelihood of benefits for a subject with the specific level of p38MAPK expression determined in (II).

**3.** The method of claim 2, wherein the function takes the form of

$$B = F( E, C ),$$

wherein
E is a matrix or profile of p38MAPK expression,
C is a matrix or profile of further relevant characteristics of the subject,
B is the probability of beneficial effects, and
F is the correlative function incorporating previous clinical experience.

**4.** The method of claim 3, wherein E is selected from the group consisting of
[ XSα ] ; [ XSα / X0α ] ; [ PSα ] ; [ PSα / P0α ] ; [ XSα ; PSα ] ;
[ XSα+XSβ+XSγ+XSδ ] ; [ (XSα+XSβ+XSγ+XSδ) / (X0α+X0β+X0γ+X0δ) ] ;
[ PSα+PSβ+PSγ+PSδ ] ; [ (PSα+PSβ+PSγ+PSδ) / (P0α+P0β+P0γ+P0δ) ] ;
[ XSα ; XSα+XSβ+XSγ+XSδ ] ; [ (XSα+XSβ+XSγ+XSδ) / (X0α+X0β+X0γ+X0δ) ] ;
[ PSα+PSβ+PSγ+PSδ ] ; and [ (PSα+PSβ+PSγ+PSδ) / (P0α+P0β+P0γ+P0δ) ] ;
wherein XAn is the expression of p38MAPKn in the tissue of interest of A, where A is either a subject S having or being suspected of having inflammatory disease, in particular rheumatoid arthritis, or a healthy control 0 not having inflammatory disease, and PAn is the level of phosphorylation of the same enzyme.

**5.** The method of claim 3 or 4, wherein C is selected from the group consisting of
[ gender ; age ; duration of disease ; blood levels of C-reactive protein ; erythrocyte sedimentation rate ; presence of erosive disease ; concomitant DMARD medication ; concomitant glucocorticoid medication ]
and any of its subsets.

**6.** The method of any of claims 1 to 5 wherein the inflammatory disease is rheumatoid arthritis.

**7.** The method of any of claims 1 to 6, wherein the inflammatory tissue is synovial tissue.

**8.** The method of claim 7, wherein the synovial tissue sample from the subject is obtained by synovectomy.

**9.** The method of any of claims 1 to 8, wherein determining p38MAPK expression comprises detecting p38MAPK protein.

**10.** The method of claim 9, wherein the p38MAPK protein is detected using an antibody against p38MAPK.

**11.** The method of claim 10, wherein the method for p38MAPK protein detection is selected from the group consisting of immunoblotting (Western blotting), immunoprecipitation and immunohistochemistry.

**12.** The method of any of claims 1 to 11, wherein determining p38MAPK expression comprises detecting p38MAPK mRNA.

**13.** The method of claim 12, wherein the method for p38MAPK mRNA detection is selected from the group consisting of hybridization (Northern blotting) and RT-PCR.

**14.** The method of any of claims 1 to 13, wherein determining p38MAPK expression comprises determination of the nucleotide sequence of p38MAPK.

**15.** The method of any of claims 1 to 14, wherein the p38MAPK is p38MAPKα.

**16.** The method of any of claims 1 to 11, wherein the p38MAPK comprises non-phosphorylated p38MAPKα and phosphorylated p38MAPKα.

**17.** The method of any of claims 1 to 16, wherein the expression of the p38MAPK is expression in the synovial lining.

**18.** The method of any of claims 1 to 16, wherein the expression of the p38MAPK is expression on macrophages.

**19.** The method of any of claims 1 to 18, wherein the inhibitor is selected from the group consisting of 4,5-diarylimizadoles, 2,4,5-triarylimidazoles, 3,4,5-triarylimidazoles, 3,4-disubstituted isoxazoles and 4,5-disubstituted isoxazoles.

**20.** The method of claim 19, wherein the inhibitor is an inhibitor of the general formula (I)

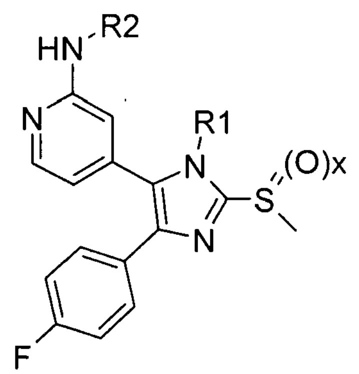

(I)

wherein
x is 0, 1 or 2;
R1 is hydrogen, methyl or $-(CH_2)_2-O-CH_3$; and
R2 is alkyl, in particular $C_1$-$C_6$ alkyl, e. g. methyl, ethyl, propyl or isopropyl; or cycloalkyl, in particular $C_3$-$C_7$ cycloalkyl, e. g. cyclopentyl or cyclohexyl; or -C(O)-R3,
wherein
R3 is alkyl, in particular $C_1$-$C_6$ alkyl, e. g. methyl, ethyl, propyl or isopropyl; or cycloalkyl, in particular $C_3$-$C_7$ cycloalkyl, e. g. cyclopentyl or cyclohexyl.

**21.** Method of classifying the clinically relevant stage of inflammatory disease in a subject having the same, which comprises

(I) obtaining an inflammatory tissue sample from the subject; and
(II) determining whether p38MAPK is expressed in the tissue sample, the expression of p38MAPK indicating whether the disease is in its early or in its established stage.

**22.** Use of the method of claim 21 for determining whether a subject having or suspected of having inflammatory disease may benefit from treatment with a p38 mitogen-activated protein kinase (p38MAPK) inhibitor.

**23.** Use of an antibody against the p38MAPK protein for determining whether a subject having or suspected of having inflammatory disease may benefit from treatment with a p38 mitogen-activated protein kinase (p38MAPK) inhibitor.

**24.** The use of claim 23, wherein the antibody is an antibody against p38MAPKα.

**25.** Use of a nucleotide sequence complementary or identical to the full length or to a part of the sequence encoding p38MAPK for determining whether a subject having or suspected of having inflammatory disease may benefit from treatment with a p38 mitogen-activated protein kinase (p38MAPK) inhibitor.

**26.** The use of claim 25, wherein the nucleotide sequence is complementary or identical to the full length or to a part of the sequence encoding p38MAPKα.

**27.** Kit for determining from an inflammatory tissue sample whether a subject having or suspected of having inflammatory

disease may benefit from treatment with a p38 mitogen-activated protein kinase (p38MAPK) inhibitor, which comprises

(I) an antibody against p38MAPK,
(II) an instruction for the use of said antibody to determine expression of the p38MAPK and thereby estimate the subject's potential benefits.

**28.** Use of a kit for determining p38MAPK expression for estimating whether a subject having or suspected of having inflammatory disease may benefit from treatment with a p38 mitogen-activated protein kinase (p38MAPK) inhibitor.

Fig. 1

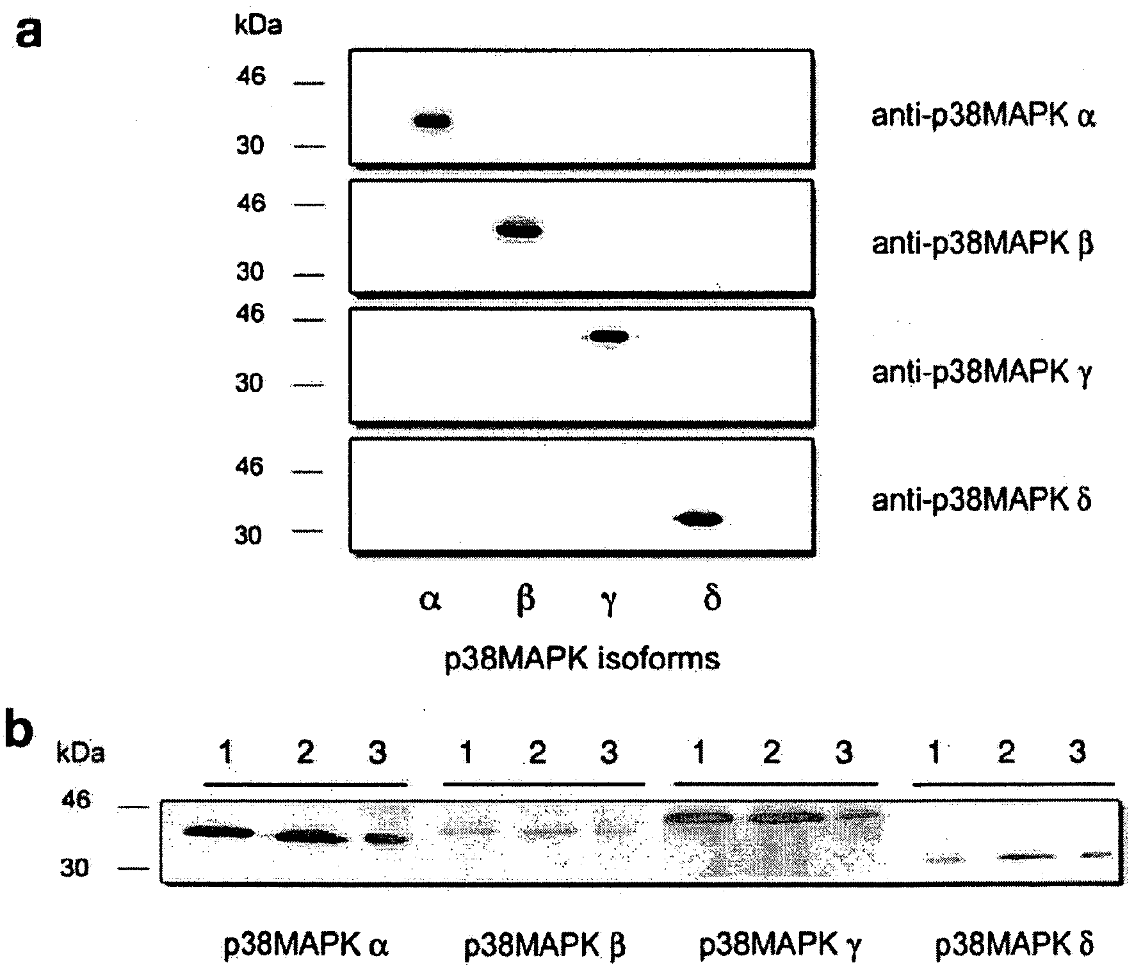
a
kDa
46
30
anti-p38MAPK α
46
30
anti-p38MAPK β
46
30
anti-p38MAPK γ
46
30
anti-p38MAPK δ
α β γ δ
p38MAPK isoforms
b
kDa
1 2 3 1 2 3 1 2 3 1 2 3
46
30
p38MAPK α
p38MAPK β
p38MAPK γ
p38MAPK δ

Fig. 2

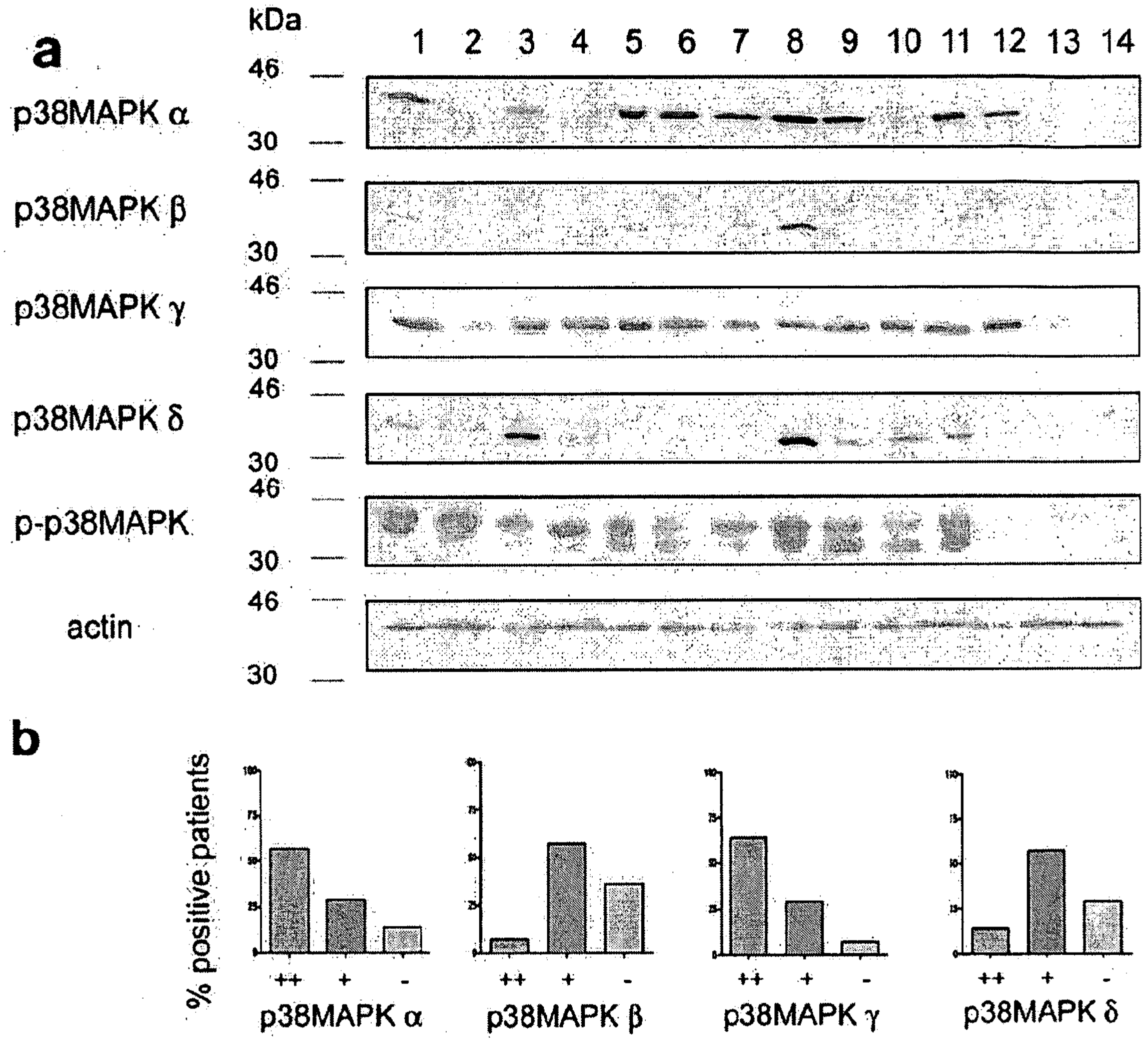
a
kDa
1 2 3 4 5 6 7 8 9 10 11 12 13 14
46
p38MAPK α
30
46
p38MAPK β
30
46
p38MAPK γ
30
46
p38MAPK δ
30
46
p-p38MAPK
30
46
actin
30
b
% positive patients
++ + -
p38MAPK α
++ + -
p38MAPK β
++ + -
p38MAPK γ
++ + -
p38MAPK δ

Fig. 3

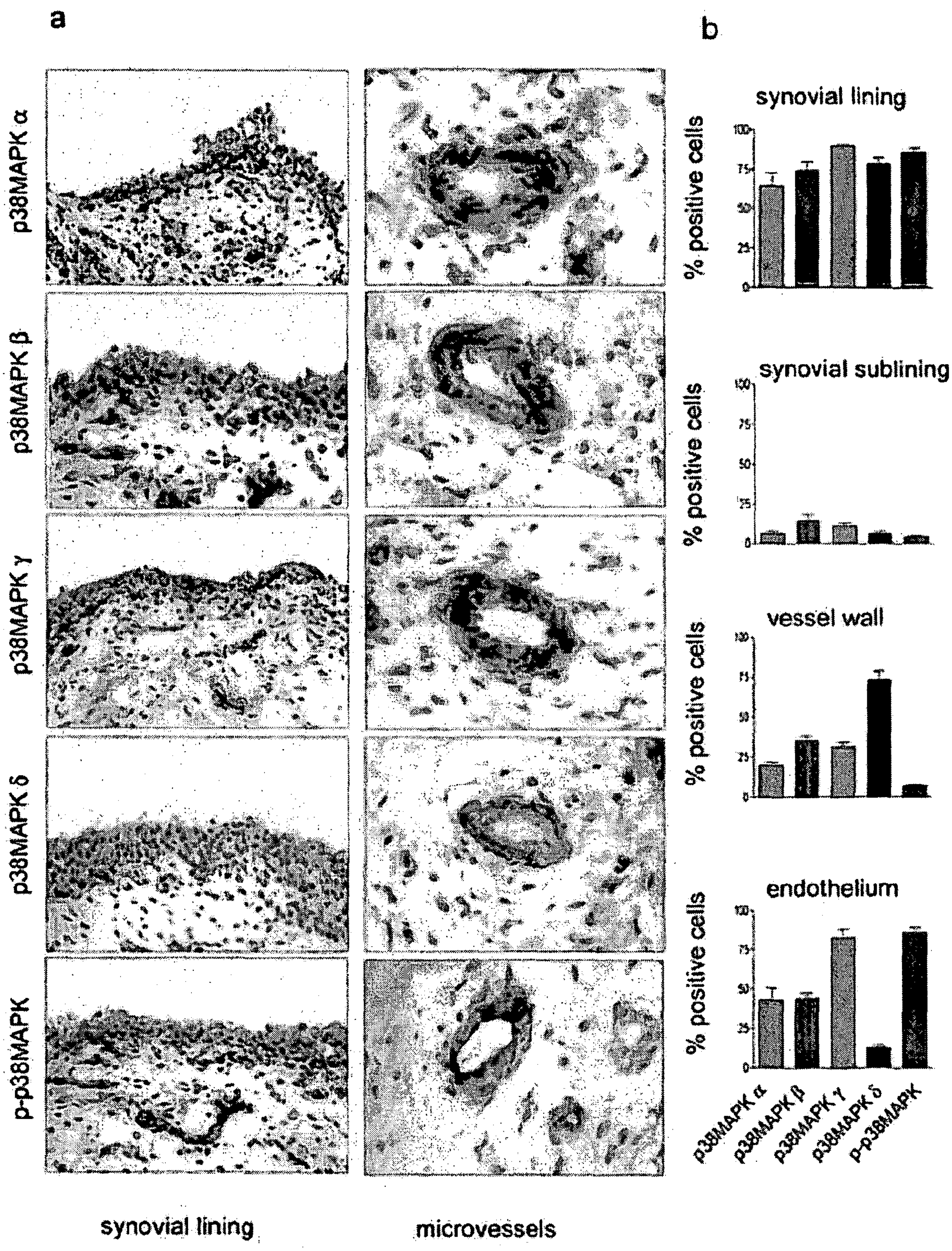
a
b
p38MAPK α
p38MAPK β
p38MAPK γ
p38MAPK δ
p-p38MAPK
synovial lining
synovial sublining
vessel wall
endothelium
% positive cells
0
25
50
75
100
p38MAPK α
p38MAPK β
p38MAPK γ
p38MAPK δ
p-p38MAPK
synovial lining
microvessels

European Patent Office

# EUROPEAN SEARCH REPORT

Application Number
EP 07 01 9573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROPERT C.: "Translating MAPK inhibitors to anti-inflammatory compounds" CURRENT ENZYME INHIBITION, vol. 1, no. 1, January 2005 (2005-01), pages 75-84, XP002467116 BENTHAM SCIENCE PUBL. * pages 78-79; figure 2 * ----- | 1-26 | INV. G01N33/50 |
| X | KAMINSKA ET AL: "MAPK signalling pathways as molecular targets for anti-inflammatory therapy-from molecular mechanisms to therapeutic benefits" BIOCHIMICA ET BIOPHYSICA ACTA (BBA) - PROTEINS & PROTEOMICS, ELSEVIER, vol. 1754, no. 1-2, 30 December 2005 (2005-12-30), pages 253-262, XP005214214 ISSN: 1570-9639 * page 257 - page 262; figures 1,2 * ----- | 1-28 | |
| X | WO 03/000189 A (BAYLOR COLLEGE MEDICINE [US]; OSBORNE C KENT [US]; SCHIFF RACHEL [US];) 3 January 2003 (2003-01-03) * claims 1-36 * ----- | 27,28 | TECHNICAL FIELDS SEARCHED (IPC) |
| | | | G01N |
| Y | SAKLATVALA JEREMY: "The p38 MAP kinase pathway as a therapeutic target in inflammatory disease" CURRENT OPINION IN PHARMACOLOGY, vol. 4, no. 4, August 2004 (2004-08), pages 372-377, XP002467115 ISSN: 1471-4892 * the whole document * ----- | 1-28 | |
| | -/-- | | |

The present search report has been drawn up for all claims

| Place of search | Date of completion of the search | Examiner |
|---|---|---|
| Munich | 5 February 2008 | Stachowiak, Olaf |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 07 01 9573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TENHUNEN OLLI ET AL: "Identification of cell cycle regulatory and inflammatory genes as predominant targets of p38 mitogen-activated protein kinase in the heart." CIRCULATION RESEARCH 1 SEP 2006, vol. 99, no. 5, 1 September 2006 (2006-09-01), pages 485-493, XP002467117 ISSN: 1524-4571 * abstract * ----- | 1-28 | |
| | | | TECHNICAL FIELDS SEARCHED (IPC) |

The present search report has been drawn up for all claims

| Place of search | Date of completion of the search | Examiner |
|---|---|---|
| Munich | 5 February 2008 | Stachowiak, Olaf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

**ANNEX TO THE EUROPEAN SEARCH REPORT ON EUROPEAN PATENT APPLICATION NO.** EP 07 01 9573

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2008

| Patent document cited in search report | Publication date | Patent family member(s) | Publication date |
|---|---|---|---|
| WO 03000189 A | 03-01-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4585771 A, Klose
- WO 8801167 A, Bender
- US 4461298 A
- US 4528298 A, Niedballa & Böttcher
- US 4402960 A, Niedballa & Böttcher
- US 4461770 A, Ferrini
- US 4608382 A, Ferrini
- US 4584310 A, Ferrini
- DE 19842833
- WO 0017192 A, Laufer
- WO 9500501 A
- WO 9903837 A, Wachter
- WO 9314081 A, Adams
- US 4440776 A, Niedballa & Böttcher
- US 4355039 A, Niedballa & Böttcher
- US 4269847 A, Niedballa & Böttcher
- WO 9603387 A, Weier
- WO 02066458 A, Laufer
- WO 03097633 A, Laufer
- WO 2004018458 A, Laufer
- EP 0004648 A, Ferrini
- EP 0005545 A, Niedballa & Böttcher
- EP 372445 A, Billheimer
- US 5364875 A, Wilde
- US 4190666 A, Cherkofsky
- GB 1155580 A
- JP 01010467 A
- SU 1415725, Vinogradow
- WO 2004110990 A
- US 4816397 A, Boss


### Non-patent literature cited in the description


- **WAGNER et al.** *Arch. Pharm. Pharm. Med. Chem.,* 2000, vol. 333 (1), 97
- **BOEHM et al.** *J. Med. Chem.,* 1996, vol. 39, 3929-3937
- **LIVERTON et al.** *J. Med. Chem.,* 1999, vol. 42, 2180-2190
- **MUSTAFA et al.** *J. prakt. Chem.,* 1972, vol. 314, 785-792
- **GUPTA et al.** *Indian J. Chem.,* 1982, vol. 22B (3), 268-269
- **WILDE et al.** *Bioorganic & Medicinal Chem. Lett.,* 1995, vol. 5 (2), 177-180
- **SALAMA et al.** *Phosphorus & Sulfur,* 1988, vol. 135 (1-2), 83-88
- **BEACH et al.** *Arch. Biochem. Biophys.,* 1992, vol. 297, 258-264
- **MADUSKUIE et al.** *J. Med. Chem.,* 1995, vol. 38, 1067-1083
- **MIOSTOŃ et al.** *Helv. Chim. Acta,* 1998, vol. 81, 1585-1595
- **MIOSTOŃ et al.** *Helv. Chim. Acta,* 1999, vol. 82, 290-296
- **LAUFER et al.** *Angew. Chem. Int. Ed.,* 2002, vol. 41 (13), 2290-2293
- **LAUFER et al.** *Chem. Med. Chem.,* 2006, vol. 1, 197-206
- **LAUFER et al.** *Chem. Med. Chem.,* 2006, vol. 2, 197-207
- **CONNOR J ; NALEBUFF EA.** *Curr. Opin. Rheumatol.,* 1995, vol. 7, 120-124
- **TAYLOR, L.D et al.** *Nucl. Acids Res.,* 1992, vol. 20, 6287-6295
- Molecular Cloning; A Laboratory Manual. Cold Spring Harbor, 1989
- Current Protocols in Molecular Biology. Greene Publishing Associates, 1989
- **BALBONI et al.** *Annu. Rev. Immunol.,* 23 April 2006, vol. 24, 391-418
- **MACBEATH et al.** *Science,* 2000, vol. 289 (5485), 1760-1763
- **HUANG ; CHEN.** *Biosens. Bioelectron.,* 08 March 2006
- **KATUS et al.** *Clin. Chem.,* March 1992, (3), 386-393

SEQUENCE LISTING

<110> c-a-i-r biosciences GmbH

<120> Method for subgroup analysis in subjects having or being suspected of having inflammatory disease, use of anti-p38MAPK antibodies, kits and their use

<130> M/47056

<150> US 60/849,784
<151> 2006-10-06

<160> 1

<170> PatentIn version 3.3

<210> 1
<211> 360
<212> PRT
<213> Homo sapiens

<400> 1

```
Met Ser Gln Glu Arg Pro Thr Phe Tyr Arg Gln Glu Leu Asn Lys Thr
1               5                   10                  15

Ile Trp Glu Val Pro Glu Arg Tyr Gln Asn Leu Ser Pro Val Gly Ser
            20                  25                  30

Gly Ala Tyr Gly Ser Val Cys Ala Ala Phe Asp Thr Lys Thr Gly Leu
        35                  40                  45

Arg Val Ala Val Lys Lys Leu Ser Arg Pro Phe Gln Ser Ile Ile His
    50                  55                  60

Ala Lys Arg Thr Tyr Arg Glu Leu Arg Leu Leu Lys His Met Lys His
65                  70                  75                  80

Glu Asn Val Ile Gly Leu Leu Asp Val Phe Thr Pro Ala Arg Ser Leu
                85                  90                  95

Glu Glu Phe Asn Asp Val Tyr Leu Val Thr His Leu Met Gly Ala Asp
            100                 105                 110

Leu Asn Asn Ile Val Lys Cys Gln Lys Leu Thr Asp Asp His Val Gln
        115                 120                 125

Phe Leu Ile Tyr Gln Ile Leu Arg Gly Leu Lys Tyr Ile His Ser Ala
    130                 135                 140

Asp Ile Ile His Arg Asp Leu Lys Pro Ser Asn Leu Ala Val Asn Glu
145                 150                 155                 160
```

```
Asp Cys Glu Leu Lys Ile Leu Asp Phe Gly Leu Ala Arg His Thr Asp
                165                 170                 175

Asp Glu Met Thr Gly Tyr Val Ala Thr Arg Trp Tyr Arg Ala Pro Glu
            180                 185                 190

Ile Met Leu Asn Trp Met His Tyr Asn Gln Thr Val Asp Ile Trp Ser
        195                 200                 205

Val Gly Cys Ile Met Ala Glu Leu Leu Thr Gly Arg Thr Leu Phe Pro
    210                 215                 220

Gly Thr Asp His Ile Asn Gln Leu Gln Gln Ile Met Arg Leu Thr Gly
225                 230                 235                 240

Thr Pro Pro Ala Tyr Leu Ile Asn Arg Met Pro Ser His Glu Ala Arg
                245                 250                 255

Asn Tyr Ile Gln Ser Leu Thr Gln Met Pro Lys Met Asn Phe Ala Asn
            260                 265                 270

Val Phe Ile Gly Ala Asn Pro Leu Ala Val Asp Leu Leu Glu Lys Met
        275                 280                 285

Leu Val Leu Asp Ser Asp Lys Arg Ile Thr Ala Ala Gln Ala Leu Ala
    290                 295                 300

His Ala Tyr Phe Ala Gln Tyr His Asp Pro Asp Asp Glu Pro Val Ala
305                 310                 315                 320

Asp Pro Tyr Asp Gln Ser Phe Glu Ser Arg Asp Leu Leu Ile Asp Glu
                325                 330                 335

Trp Lys Ser Leu Thr Tyr Asp Glu Val Ile Ser Phe Val Pro Pro Pro
            340                 345                 350

Leu Asp Gln Glu Glu Met Glu Ser
        355                 360
```